# EUROPÄISCHE PATENTANMELDUNG

(11) **EP 1 813 595 A1**
(43) Veröffentlichungstag der Anmeldung: **01.08.2007**
(21) Anmeldenummer: 07000856.0
(22) Anmeldetag: 17.01.2007
(51) Int. Cl.: C07C 209/26, C07C 211/19

(54) **"3(4),7(8)-Bis (aminomethyl)-bicyclo[4.3.0] nonan und ein Verfahren zu seiner Herstellung**

(30) Priorität: 31.01.2006 DE 10604324
(71) Anmelder: OXEA Deutschland GmbH, 46147 Oberhausen (DE)
(72) Erfinder: Bavaj, Paolo, 60320 Frankfurt (DE); Springer, Helmut, 46539 Dinslaken (DE)

(57) **Zusammenfassung**

Die vorliegende Erfindung betrifft die chemische Verbindung 3(4),7(8)-Bis(aminomethyl)-bicyclo[4.3.0]nonan sowie ein Verfahren zu ihrer Herstellung, wobei man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bisformyl-bicyclo[4.3.0]nonan reduktiv aminiert.

## Beschreibung

Die vorliegende Erfindung betrifft die chemische Verbindung 3(4),7(8)-Bis(aminomethyl)-bicyclo[4.3.0]nonan und ein Verfahren zu ihrer Herstellung aus Bicyclo[4.3.0]nona-3,7-dien.

Kondensierte, alicyclische, ungesättigte Kohlenwasserstoffe mit isolierten Doppelbindungen in den Ringen sind wertvolle Ausgangsprodukte, die sich zu anwendungstechnisch wichtigen Verbindungen umsetzen lassen. Das ringförmig aufgebaute und kondensierte Kohlenwasserstoffgerüst verleiht dabei besondere Eigenschaften. Ein wichtiges Beispiel für diese Verbindungsklasse ist das durch Dimerisierung von Cyclopentadien leicht zugängliche und auch großtechnisch hergestellte Dicyclopentadien (DCP), das sich zu anwendungstechnisch wichtigen Verbindungen umsetzen lässt, denen das Tricyclodecan-Gerüst besondere Eigenschaften verleiht. Die vom DCPabgeleiteten Verbindungen mit Tricyclodecan-Struktur werden in der Literatur häufig auch als TCD-Derivate bezeichnet (Chemiker-Zeitung, 98, 1974, Seiten 70 bis 76).

Insbesondere die Hydroformylierung von DCP liefert interessante TCD-Aldehyde, wie 3(4),8(9)-Bisformyl-tricyclo[5.2.10^{2.6}]decan, auch als TCD-Dialdehyd bezeichnet, das zu wichtigen Zwischenprodukten weiterverarbeitet wird.

Wegen ihrer thermischen Labilität, die bei der destillativen Aufarbeitung zu Verlusten führt, wird TCD-Dialdehyd zumeist nicht rein isoliert, sondern als Rohprodukt der Hydroformylierungsreaktion weiterverarbeitet. So führt die reduktive Aminierung von TCD-Dialdehyd zu TCD-Diamin {3(4),8(9)-Bis(aminomethyl)-tricyclo[5.2.1.0^{2.6}]decan}, das als wertvolles Zwischenprodukt in zahlreichen technisch ausgeübten Synthesen Verwendung findet, beispielsweise zur Herstellung von lichtbeständigen Polyurethansystemen gemäß DE 28 19 980 A1, zur Herstellung von zahntechnischen Materialien (WO2002/013767 A2, EP 678 533 A2), zur Synthese von Polyamiden (EP 168 816 A2), polyfunktionellen Epoxidharzsystemen (JP 58 135 875 A), hitzehärtbaren Beschichtungsmaterialien (EP 59 962 A1), von Epoxidharzhärtern (JP 54 004 992 A) und zur Herstellung von TCD-Diisocyanat (NL 66 14 717 A).

Die Herstellung von Aldehyden durch katalytische Anlagerung von Kohlenmonoxid und Wasserstoff an olefinische Doppelbindungen ist bekannt. Während diese Umsetzung früher nahezu ausschließlich mit Kobalt als Katalysator durchgeführt wurde, arbeiten moderne Verfahren mit metallischem Rhodium oder mit Rhodiumverbindungen als Katalysatoren, die allein oder mit komplexbildenden Liganden, z.B. organischen Phosphinen oder Estern der phosphorigen Säure, eingesetzt werden. Unter den Reaktionsbedingungen als Katalysator wirksam sind nach übereinstimmender Auffassung der Fachwelt Hydridocarbonylverbindungen des Rhodiums, die sich durch die allgemeine Formel H[Rh(CO)₄₋ₓLₓ] wiedergeben lassen, wobei L einen Liganden bezeichnet und x gleich 0 oder eine ganze Zahl von 1 bis 3 ist.

Einen Sonderfall stellt die Hydroformylierung von Dienen dar. Während bei der Hydroformylierung konjugierter Diene unter den üblichen Bedingungen der Oxo-Synthese fast ausschließlich Monoaldehyde erhalten werden, lassen sich aus Dicyclopentadien (DCP) mit seinen isolierten Doppelbindungen neben den Mono- auch die Disubstitutionsprodukte gewinnen. Aufgrund der großen Bedeutung der Hydroformylierungsprodukte des DCP finden sich auch in der technischen Literatur zahlreiche Arbeiten, die sich sowohl mit der Hydroformylierungsreaktion von DCP als auch mit der nachfolgenden Aufarbeitung des Rohproduktes befassen. So behandeln DE 38 22 038 A1 und GB 1 170 226 die Hydroformylierung von DCP in Gegenwart von Rhodium in einem organischen Lösungsmittel bei erhöhtem Druck und erhöhter Temperatur. Eine zusammenfassende Darstellung über die Hydroformylierung von Dicyclopentadien findet sich in der Chemiker-Zeitung 98, 1974, 70-76, wobei ebenfalls auf die thermische Labilität der TCD-Aldehyde hingewiesen wird, die bei der destillativen Aufarbeitung des rohen Hydroformylierungsgemisches zu hohen Produktverlusten führt. Daher werden TCD-Dialdehyde zumeist nicht rein isoliert, sondern in ihren Gemischen mit den Nebenprodukten der Oxo-Synthese weiterverarbeitet. Es finden sich im Stand der Technik, z.B. in EP-1 065 194 A1 oder US 5,138,101 A aber auch Hinweise auf extraktive Aufarbeitungsprozesse ohne thermische Belastung. Dabei wird das organische Rohgemisch mit einem polaren organischen Lösungsmittel, beispielsweise mit einem mehrwertigen Alkohol oder mit einem Methanol/Wasser-Gemisch extrahiert, wobei die TCD-Dialdehyde in die polare, alkoholische Phase übergehen und der Hydroformylierungskatalysator in der Kohlenwasserstoffphase verbleibt.

Die Herstellung von TCD-Diamin ist aus DE 38 22 038 A1 bekannt. Danach wird Dicyclopentadien in organischer Lösung in Gegenwart von Rhodium zum rohen TCD-Dialdehyd hydroformyliert, das anschließend ohne weitere Abtrennung des TCD-Dialdehyds und ohne Entfernung des Katalysators in einem zweiten Reaktionsschritt in Gegenwart eines primären Amins reduktiv aminiert wird.

Nach der JP 10 087 573 A erfolgt die Herstellung von TCD-Diamin durch Hydrierung des entsprechenden TCD-Dialdehyddioxims in Gegenwart von Fe-Cr-modifiziertem Raney-Nickel.

Aufgrund der großen wirtschaftlichen Bedeutung, die aminomethylierte Diamine auf Basis von kondensierten, alicyclischen Kohlenwasserstoffen besitzen, besteht daher der Bedarf nach der Bereitstellung weiterer, preisgünstig verfügbarer aminomethylierter Diamine in hoher Reinheit, die ein ringförmig aufgebautes Kohlenwasserstoffgerüst mit kondensierten Ringen aufweisen.

Die vorliegende Erfindung betrifft daher die chemische Verbindung 3(4),7(8)-Bis(aminomethyl)-bicyclo[4.3.0]nonan.

Ebenfalls besteht die Erfindung in einem Verfahren zur Herstellung von 3(4),7(8)-Bis(aminomethyl)-bicyclo[4.3.0]nonan durch Hydroformylierung von Bicyclo[4.3.0]nona-3,7-dien mit nachfolgender reduktiver Aminierung. Es ist dadurch gekennzeichnet, dass man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160°C und Drücken von 5 bis 35 MPa mit Synthesegas umsetzt und das so erhaltene 3(4),7(8)-Bis-formyl-bicyclo[4.3.0]nonan anschließend zum 3(4),7(8)-Bis(aminomethyl)-bicyclo[4.3.0]nonan reduktiv aminiert.

Die erfindungsgemäße Verbindung leitet sich von Bicyclo[4.3.0]nona-3,7-dien ab, das technisch durch Diels-Alder-Reaktion von Butadien mit Cyclopentadien hergestellt wird und das daher in preisgünstigen Mengen zur Verfügung steht.

Die Zählung der in dem ungesättigten, bicyclischen Kohlenwasserstoff gebundenen Kohlenstoffatome erfolgt nach folgender Reihenfolge: 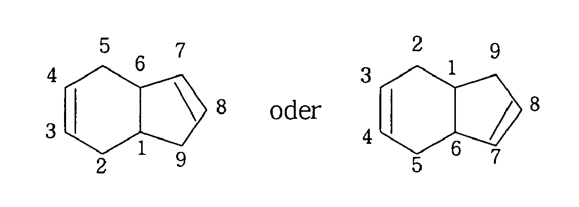 wobei beide Strukturformeln identisch sind.

Bei der erfindungsgemäßen Verbindung 3(4),7(8)-Bis(aminomethyl)-bicyclo[4.3.0]nonan handelt es sich um ein Gemisch aus verschiedenen Isomeren des Bis(aminomethyl)-bicyclo[4.3.0]nonans, bei denen die Aminomethylgruppe im Sechsring einmal an der 3- oder an der 4-Position und die Aminomethylgruppe im Fünfring einmal an der 7- oder an der 8-Position gebunden sein kann.

In Analogie zu der bei den TCD-Derivaten gemäß Chemiker-Zeitung, 98, 1974, Seiten 70 bis 76 üblichen Schreibweise kann die erfindungsgemäße Verbindung 3(4),7(8)-Bis(aminomethyl)-bicyclo[4.3.0]nonan formelmäßig wie folgt beschrieben werden: 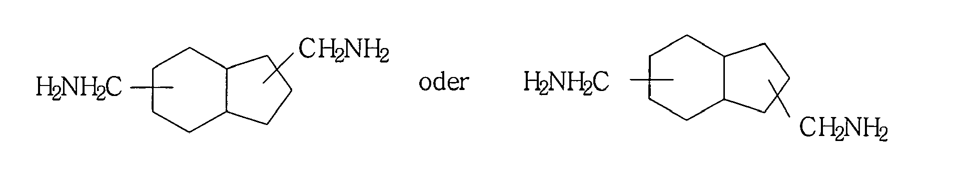 wobei beide Strukturformeln identisch sind.

Das Ausgangsprodukt Bicyclo[4.3.0]nona-3,7-dien kann als solches oder in Lösung der Hydroformylierung zugeführt werden. Geeignete Lösungsmittel sind solche, in denen Ausgangsmaterial, Reaktionsprodukt und Katalysator löslich sind und die sich unter den Reaktionsbedingungen inert verhalten, beispielsweise wasserunlösliche Ketone, Dialkylether, aliphatische Nitrile, aromatische Kohlenwasserstoffe wie Benzol, Toluol, die isomeren Xylole oder Mesitylen und gesättigte cycloaliphatische Kohlenwasserstoffe wie Cyclopentan oder Cylcohexan oder gesättigte aliphatische Kohlenwasserstoffe, wie n-Hexan, n-Heptan oder n-Octan. Der Anteil des Lösungsmittels im Reaktionsmedium kann über einen weiten Bereich variiert werden und beträgt üblicherweise zwischen 10 und 80 Gew.-%, vorzugsweise 20 bis 50 Gew.-%, bezogen auf das Reaktionsgemisch.

Die Hydroformylierungsstufe führt man in einem homogenen Reaktionssystem durch. Der Begriff homogenes Reaktionssystem steht für eine im wesentlichen aus Lösungsmittel, falls in der Reaktionsstufe zugesetzt, Katalysator, überschüssiger Organophosphorverbindung, unumgesetzter Ausgangsverbindung und Hydroformylierungsprodukt zusammengesetzte homogene Lösung.

Als Katalysatoren verwendet man Übergangsmetallverbindungen der Gruppe VIII des Periodensystems der Elemente, die Organophosphorverbindungen in komplexer Bindung enthalten. Vorzugsweise setzt man Komplexverbindungen des Kobalts, Rhodiums, Iridiums, Nickels, Eisens, Platins, Palladiums oder Rutheniums und insbesondere des Kobalts, Rhodiums und Iridiums ein. Besonders bevorzugt ist die Verwendung von Rhodium-Komplexverbindungen, die organische Phosphor(III)-Verbindungen als Liganden enthalten. Derartige Komplexverbindungen und ihre Herstellung sind bekannt (z.B. aus US 3 527 809 A, US 4 148 830 A, US 4 247 486 A, US 4 283 562 A). Sie können als einheitliche Komplexverbindungen oder auch als Gemisch unterschiedlicher Komplexverbindungen eingesetzt werden. Die Rhodium-Konzentration im Reaktionsmedium erstreckt sich über einen Bereich von 5 bis 1000 Gew.-ppm und beträgt vorzugsweise 10 bis 700 Gew.-ppm. Insbesondere wendet man Rhodium in Konzentrationen von 20 bis 500 Gew.-ppm, jeweils bezogen auf das homogene Reaktionsgemisch an. Die Hydroformylierung wird in Gegenwart eines Katalysatorsystems aus Rhodium-Organophosphor-Komplexverbindung und freiem, d.h. überschüssigen Organophosphor-Liganden durchgeführt, der mit Rhodium keine Komplexverbindung mehr eingeht. Der freie Organophosphor-Ligand kann der gleiche sein wie in der Rhodium-Komplexverbindung, es können aber auch von diesem verschiedene Liganden eingesetzt werden. Der freie Ligand kann eine einheitliche Verbindung sein oder aus einem Gemisch verschiedener Organophosphorverbindungen bestehen. Beispiele für Rhodium-Organophosphor-Komplexverbindungen, die als Katalysatoren Anwendung finden können, sind in US 3 527 809 A beschrieben. Zu den bevorzugten Liganden in den Rhodium-Komplexkatalysatoren zählen z. B. Triarylphosphine wie Triphenylphosphin, Trialkylphosphine wie Tri-(n-octyl)-phosphin, Trilaurylphosphin, Tri-(cyclohexyl)-phosphin, Alkylarylphosphine, Alkylphosphite, Arylphosphite, Alkyldiphosphite und Aryldiphosphite. So können Rhodium-Komplexverbindungen, die Arylphosphite der allgemeinen Formel P(OR¹)(OR²)(OR³) komplexgebunden enthalten, wobei wenigstens eine der Gruppen R¹, R² oder R³ für einen in ortho-Position substituierten Phenylring steht, ebenfalls eingesetzt werden. Als geeignete Komplexliganden haben sich Tris(2-tertiär-butylphenyl)phosphit oder Tris(2-tertiär-butyl-4-methylphenyl)phosphit erwiesen. Die Rhodium katalysierte Hydroformylierung von Olefinen mit Phosphit modifizierten Komplexverbindungen ist aus EP 0 054 986 A1 bekannt. Wegen seiner leichten Zugänglichkeit wird Triphenylphosphin besonders häufig angewandt.

Üblicherweise beträgt in der homogenen Reaktionsmischung das molare Verhältnis von Rhodium zu Phosphor 1 : 5 bis 1 : 200, jedoch kann der molare Anteil des Phosphors in Form organischer Phosphorverbindungen auch höher sein. Vorzugsweise setzt man Rhodium und organisch gebundenen Phosphor in molaren Verhältnissen von 1 : 10 bis 1 : 100 ein.

Verwendet man in der Hydroformylierungsstufe ein anderes Übergangsmetall der Gruppe VIII des Periodensystems der Elemente als Rhodium, so liegt die Konzentration an Übergangsmetall und das molare Verhältnis von Übergangsmetall zu Phosphor in den Bereichen, die man auch bei Rhodium wählt. Die jeweiligen optimalen Werte lassen sich in Abhängigkeit von dem jeweils eingesetzten Übergangsmetall durch einfache Routineversuche ermitteln.

Die Bedingungen, unter denen die Hydroformylierung abläuft, können innerhalb weiter Grenzen variieren und den individuellen Gegebenheiten angepasst werden. Sie hängen u.a. vom Einsatzmaterial, vom ausgewählten Katalysatorsystem und vom angestrebten Umsetzungsgrad ab. Üblicherweise führt man die Hydroformylierung von Bicyclo[4.3.0]nona-3,7-dien bei Temperaturen von 70 bis 160°C durch. Bevorzugt hält man Temperaturen von 80 bis 150°C und insbesondere von 90 bis 140°C ein. Der Gesamtdruck erstreckt sich über einen Bereich von 5 bis 35 MPa, vorzugsweise 10 bis 30 MPa und insbesondere 20 bis 30 MPa. Das molare Verhältnis von Wasserstoff zu Kohlenmonoxid bewegt sich üblicherweise zwischen 1:10 und 10:1, Mischungen, die Wasserstoff und Kohlenmonoxid im molaren Verhältnis 3:1 bis 1:3, insbesondere etwa 1:1, enthalten, sind besonders geeignet.

Man bildet den Katalysator üblicherweise aus den Komponenten Übergangsmetall oder Übergangsmetallverbindung, Organophosphorverbindung und Synthesegas unter den Bedingungen der Hydroformylierungsreaktion im Reaktionsgemisch. Es ist aber auch möglich, den Katalysator zunächst zu präformieren und ihn anschließend der eigentlichen Hydroformylierungsstufe zuzuführen. Die Bedingungen der Präformierung entsprechen dabei im allgemeinen den Hydroformylierungsbedingungen.

Zur Herstellung des Hydroformylierungskatalysators gelangt das Übergangsmetall der Gruppe VIII des Periodensystems der Elemente, insbesondere Rhodium, entweder in metallischer Form oder als Verbindung zum Einsatz. In metallischer Form verwendet man das Übergangsmetall entweder als feinverteilte Partikel oder in dünner Schicht auf einem Träger, wie Aktivkohle, Calciumcarbonat, Aluminiumsilikat, Tonerde niedergeschlagen. Als Übergangsmetallverbindungen eignen sich Salze aliphatischer Mono- und Polycarbonsäuren, wie Übergangsmetall-2-Ethylhexanoate, -Acetate, -Oxalate, -Propionate oder -Malonate. Weiterhin können Salze anorganischer Wasserstoff- und Sauerstoffsäuren, wie Nitrate oder Sulfate, die verschiedenen Übergangsmetalloxide oder auch Übergangsmetallcarbonylverbindungen, wie Rh₃(CO)₁₂, Rh₆(CO)₁₆, CO₂(CO)₈, CO₄(CO)₁₆, Fe(CO)₅, Fe₂(CO)₉, Ir₂(CO)₈, Ir₄(CO)₁₂ oder Übergangsmetall-Komplexverbindungen, z.B. Cyclopentadienylrhodiumverbindungen, Rhodiumacetylacetonat, Cyclopentadienylkobalt-Cyclooctadien-1,5, Fe(CO)₃-Cyclooctadien-1,5, [RhCl(Cyclooctadien-1,5]₂ oder PtCl₂(Cyclooctadien-1,5) eingesetzt werden. Übergangsmetallhalogenverbindungen kommen wegen ihres korrosiven Verhaltens der Halogenidionen weniger in Betracht.

Bevorzugt werden Übergangsmetalloxide und insbesondere Übergangsmetallacetate und -2-ethylhexanoate. Als besonders geeignet haben sich Rhodiumoxid, Rhodiumacetat, Rhodium-2-ethylhexanoat, Kobaltoxid, Kobaltacetat und Kobalt-2-ethylhexanoat erwiesen.

Die Hydroformylierungsstufe kann sowohl absatzweise als auch kontinuierlich durchgeführt werden. Nach dem erfindungsgemäßen Verfahren wird das Ausgangsolefin Bicyclo[4.3.0]nona-3,7-dien nahezu vollständig umgesetzt und es wird ein rohes Hydroformylierungsprodukt mit einem hohen Gehalt an dem gewünschten Bisformylprodukt erhalten, der im allgemeinen über 75 Gew.-%, bezogen auf das rohe Hydroformylierungsprodukt, liegt.

Das Umsetzungsprodukt der Hydroformylierungsstufe wird ohne weitere Reinigung und ohne Katalysatorabtrennung reduktiv aminiert.

Unter reduktiver Aminierung versteht man die Reaktion des 3(4),7(8)-Bisformyl-bicyclo[4.3.0]nonans mit Wasserstoff und Ammoniak in Gegenwart eines Hydrierkatalysators. Man kann aber auch den Dialdehyd zunächst mit einem primären Amin unter Bildung eines Diazomethins umsetzen und daraufhin das Diazomethin mit Wasserstoff und Ammoniak in Gegenwart eines Hydrierkatalysators behandeln. Auch diese Reaktion wird im Sinne der Erfindung als reduktive Aminierung bezeichnet.

Zur Bildung des Diazomethins gibt man zu dem nicht aufgearbeiteten Hydroformylierungsgemisch je mol 3(4),7(8)-Bisformyl-bicyclo[4.3.0]nonan 2 bis 6 mol, insbesondere 3 bis 4 mol eines primären Amins. Die Reaktion zwischen den Ausgangsstoffen läuft bereits bei Raumtemperatur ab, sie kann durch Erhitzen auf 20 bis 60°C, insbesondere 30 bis 50°C beschleunigt werden. Als primäre Amine geeignet sind Amine mit 2 bis 10 Kohlenstoffatomen im Molekül. Mit besonderem Erfolg werden Amine mit 3 bis 5 Kohlenstoffatomen im Molekül, vorzugsweise n-Butylamin, eingesetzt.

Die reduktive Aminierung des Dialdehyds oder des Diazomethins wird zweckmäßig bei Temperaturen von 60 bis 150°C, vorzugsweise 80 bis 140°C durchgeführt. Der Wasserstoffdruck im Reaktionsgefäß beträgt bei der Reaktionstemperatur 2 bis 12 und insbesondere 8 bis 10 MPa.

Als Hydrierkatalysatoren werden Nickel oder Kobalt verwendet, entweder in Form von Raney-Nickel bzw. Raney-Kobalt oder auch in Form entsprechender Trägerkatalysatoren. Ein bevorzugter Katalysator enthält 50 bis 60 Gew.-% Nickel auf Kieselgur.

Ammoniak soll zweckmäßigerweise im Überschuß angewendet werden. Je mol Formylgruppe bzw. je mol Diazomethingruppe sind mindestens 2 mol Ammoniak erforderlich, vorzugsweise werden 4 bis 10 mol Ammoniak eingesetzt.

Die reduktive Aminierung des Dialdehyds oder des Diazomethins kann in Abwesenheit von Lösungsmitteln durchgeführt werden, üblicherweise wirkt das Endprodukt selbst als Lösungsmittel. Bei kleinen diskontinuierlichen Ansätzen ist es jedoch vorteilhaft, in einem Lösungsmittel zu arbeiten. Besonders gute Ergebnisse werden bei Verwendung von Tetrahydrofuran, Isobutanol, Butanol oder Isopropanol als Lösungsmittel erzielt.

Das Gemisch der isomeren Diamine wird in guten Ausbeuten erhalten. Nur ein kleiner Teil der Dialdehyde geht in höhermolekulare Kondensationsprodukte über. Sie sind im Reaktionsgemisch gelöst und stören die Produktentnahme aus dem Reaktor und eine störungsfreie Aufarbeitung der Rohprodukte nicht.

Zur Abtrennung von 3(4),7(8)-Bis(aminomethyl)-bicyclo[4.3.0]nonan destilliert man das Reaktionsgemisch, vorzugsweise unter vermindertem Druck. Man erhält die Verbindungen als farblose Flüssigkeit, die beispielsweise unter einem Druck von 5 hPa bei etwa 149°C siedet. Der Hydrierkatalysator sowie das in der Hydroformylierungsstufe eingesetzte Übergangsmetall fallen im Destillationsrückstand an und werden nach bekannten Verfahren zurückgewonnen.

Das Reaktionsprodukt der Hydroformylierung von Bicyclo[4.3.0]nona-3,7-dien kann aber auch zunächst nach konventionellen Verfahren destilliert werden und als gereinigtes Produkt reduktiv aminiert werden. Überraschenderweise kann 3(4),7(8)-Bisformyl-bicyclo[4.3.0]nonan mit hoher destillativer Ausbringung in reiner Form erhalten werden. Dies ist um so mehr überraschend, weil der Stand der Technik auf die thermische Labilität von Dialdehyden mit kondensierten, alicyclischen Ringstrukturen hinweist. Übergangsmetall, vorzugsweise Rhodium und zugesetzte Organophosphorverbindung fallen im Destillationsrückstand an und werden nach bekannten Methoden zurückgewonnen. Die anschließende reduktive Aminierung des gereinigten 3(4),7(8)-Bisformyl-bicyclo[4.3.0]nonans erfolgt wie bei der Umsetzung des rohen Hydroformylierungsprodukts.

Das erfindungsgemäße Verfahren gestattet einen einfachen und kostengünstigen Zugang zu 3(4),7(8)-Bis(aminomethyl)-bicyclo[4.3.0]nonan in hoher Ausbeute und in hoher Reinheit. Das nach dem erfindungsgemäßen Verfahren hergestellte Diamin lässt sich in ausgezeichneter Weise für unterschiedliche Anwendungen einsetzen, beispielsweise als Härter für Epoxidharze, als Bestandteil in Polyurethanen sowie Polyamiden, sowie zur Herstellung von Folgeprodukten, die als Additive in Treibstoffen und Schmiermitteln verwendet werden.

Im Folgenden wird das erfindungsgemäße Verfahren näher erläutert, es ist jedoch nicht auf die beschriebenen Ausführungsformen beschränkt.

### Beispiele

### Herstellung von 3(4),7(8)-Bis(aminomethyl)-bicyclo[4.3.0]nonan

### 1. Herstellung von 3(4),7(8)-Bisformyl-bicyclo[4.3.0]nonan

In einem Stahlautoklaven mit Magnetrührer wurden 1000 g Bicyclo[4.3.0]nona-3,7-dien, technische Qualität, und 1000 g Toluol vorgelegt. Nach Zusatz von 12,75 g Triphenylphosphin sowie 50 mg Rh, in Form einer toluolischen Lösung von Rh-2-ethylhexanoat mit einem Gehalt von 7062 mg Rh/kg, wurde das Gemisch auf 130°C erwärmt und unter einem Druck von 26 MPa mit Synthesegas behandelt. Nach einer Reaktionszeit von 8 Stunden wurde die Hydroformylierungsreaktion beendet.

Die organische Phase wurde gaschromatographisch untersucht.

| GC-Analyse (Flächenprozent ohne Toluol) | |
|---|---|
| Vorlaufkomponenten | 0,2 |
| Bicyclo[4.3.0]nona-3,7-dien-Bereich | 0,1 |
| Komponenten | 4,6 |
| 3(4),7(8)-Bisformyl-bicyclo[4.3.0]nonan | 89,1 |
| Triphenylphosphin/Triphenylphosphinoxid | 1,2 |
| Hochsieder | 4,8 |

### 2. Herstellung von 3(4),7(8)-Bis(aminomethyl)-bicyclo[4.3.0]nonan

Das nach der Hydroformylierung erhaltene rohe 3(4),7(8)-Bisformyl-bicyclo[4.3.0]nonan wurde durch Destillation an einem Dünnschichtverdampfer weitgehend vom Toluol befreit (Manteltemperatur 140 °C, Druck 100 hPa). Es fiel ein Rückstand an, der nach gaschromatographischer Analyse neben 6,7 % Toluol und 83,8 % 3(4),7(8)-Bisformyl-bicyclo[4.3.0]nonan noch 9,5 % Komponenten enthielt. Der Rückstand wurde anschließend in die reduktive Aminierung eingesetzt.

Hierzu wurden 380,3 g n-Butylamin in einem 2-l Dreihalskolben mit Rührer vorgelegt und innerhalb von 120 Minuten bei einer Temperatur von 50°C mit 477,5 g Destillationsrückstand versetzt. Das Gemisch wurde noch für zwei weitere Stunden bei 50°C gerührt, danach kühlte man ab und trennte die angefallene Wasserphase (50,4 g) von der organischen Phase (807,4 g) (Schiffsche Base).

805,0 g der organischen Phase sowie 48,3 g Ni 52/35 - Katalysator der Firma Johnson-Matthey Plc - wurden danach in einem 3-l Autoklav vorgelegt. Nach Aufdrücken von Wasserstoff bis zu einem Druck von 1,5 MPa wurden 340,6 g Ammoniak zugepumpt. Das Gemisch wurde anschließend auf 125°C erwärmt und der Druck durch weitere Wasserstoffzufuhr auf 10 MPa eingestellt. Die aus der Umsetzung zwischen 3(4),7(8)-Bisformyl-bicyclo[4.3.0]nonan und n-Butylamin zuvor gebildete Schiffsche Base wurde unter diesen Bedingungen nach 6 Stunden umgesetzt. Nach Reaktionsende wurde abgekühlt, entspannt und vom Katalysator abfiltriert. Das anfallende Reaktionsprodukt wurde gaschromatographisch untersucht.

| GC-Analyse (Flächenprozent) | |
|---|---|
| Vorlaufkomponenten | 0,1 % |
| n-Butylamin | 36,2 % |
| Toluol / Methylcylcohexan 3(4),7(8)-Bis(aminomethyl)- | 4,5 % |
| bicyclo[4.3.0]nonan | 51,7 % |
| Sonstige | 7,5 % |

Zur Aufarbeitung wurden 721,7 g des rohen 3(4),7(8)-Bis(aminomethyl)-bicyclo[4.3.0]nonans an einer Kolonne mit 4,5 theoretischen Böden destilliert. Man erhielt 281,4 g Hauptfraktion in einem Siedebereich von 148-149°C bei einem Druck von 5 hPa mit folgender Zusammensetzung.

| GC-Analyse (Flächenprozent) | |
|---|---|
| Vorlaufkomponenten | <0,1 % |
| 3(4),7(8)-Bis(aminomethyl)-bicyclo[4.3.0]nonan | 99,3 % |
| Sonstige | 0,7 % |

Die Gesamtausbeute an 3(4),7(8)-Bis(aminomethyl)-bicyclo[4.3.0]nonan beträgt über alle Stufen 73,0 % d.Th., bezogen auf Bicyclo[4.3.0]nona-3,7-dien.

### Charakterisierung:

NMR-Daten
¹H-NMR (500 MHz, DMSO-d₆, ppm): 0,57-2,71 (m, 22 H, CH, CH₂ und NH₂)
¹³C-NMR (125 MHz, DMSO-d₆, ppm): 24,48-49,63 (CH und CH₂)
GC-MS (PCl): m/z = 365 {2M·H}⁺, 239 {M·C₄H₉}⁺, 183 {M·H}⁺
IR-Daten (Diamant-ATR-IR Spektroskopie)
ν (cm⁻¹): 3369(w), 3287 (w), 2908 (s), 2852 (s), 1603 (m, br), 1446 (m), 807 (s, br)
Dichte bei 20°C 0,9845 g/cm³
Brechzahl n_{D} bei 20°C 1,5106

Das erfindungsgemäße Verfahren eröffnet einen eleganten Herstellungsweg für 3(4),7(8)-Bis(aminomethyl)-bicyclo[4.3.0]nonan in hohen Ausbeuten. Die neue Verbindung 3(4),7(8)-Bis(aminomethyl)-bicyclo[4.3.0]nonan weist eine alicyclische Ringstruktur mit kondensierten Ringen auf, die sich hervorragend als Härter für Epoxidharze oder als Bestandteil für Polyurethane und Polyamide eignet. Ebenfalls kann sie zur Herstellung von Folgeprodukten verwendet werden, die als Additive in Treibstoffen und Schmiermitteln zum Einsatz kommen.

## Patentansprüche

1. Verfahren zur Herstellung von 3(4),7(8)-Bis(aminomethyl)-bicyclo[4.3.0]nonan durch Hydroformylierung von Bicyclo[4.3.0]nona-3,7-dien mit nachfolgender reduktiver Aminierung, **dadurch gekennzeichnet, dass** man Bicyclo[4.3.0]nona-3,7-dien in homogener organischer Phase in Gegenwart von Organophosphorverbindungen in komplexer Bindung enthaltenden Übergangsmetallverbindungen der Gruppe VIII des Periodensystems der Elemente und überschüssiger Organophosphorverbindung bei Temperaturen von 70 bis 160 °C und Drücken von 5 bis 35 MPa mit Synthesegas umsetzt und das so erhaltene 3(4),7(8)-Bisformyl-bicyclo[4.3.0]nonan anschließend zum 3(4),7(8)-Bis(aminomethyl)-bicyclo[4.3.0]nonan reduktiv aminiert.

2. Verfahren gemäß Anspruch 1, **dadurch gekennzeichnet, dass** man als Organophosphorverbindungen organische Phosphor(III)-Verbindungen ausgewählt aus der Gruppe von Triarylphosphinen, Trialkylphosphinen, Alkylarylphosphinen, Alkylphosphiten, Arylphosphiten, Alkyldiphosphiten oder Aryldiphosphiten einsetzt.

3. Verfahren nach Anspruch 2, **dadurch gekennzeichnet, dass** man als Triarylphosphin Triphenylphosphin und als Arylphosphit Tris(2-tertiär-butylphenyl)phosphit oder Tris(2-tertiär-butyl-4-methylphenyl)phosphit einsetzt.

4. Verfahren gemäß einem oder mehreren der Ansprüche 1 bis 3, **dadurch gekennzeichnet, dass** als Übergangsmetallverbindungen der Gruppe VIII des Periodensystems der Elemente Verbindungen von Rhodium, Kobalt, Iridium, Nickel, Palladium, Platin, Eisen oder Ruthenium verwendet werden.

5. Verfahren gemäß einem oder mehreren der Ansprüche 1 bis 4, **dadurch gekennzeichnet, dass** als Übergangsmetallverbindungen der Gruppe VIII des Periodensystems der Elemente Verbindungen von Rhodium eingesetzt werden.

6. Verfahren gemäß einem oder mehreren der Ansprüche 1 bis 5, **dadurch gekennzeichnet, dass** man Rhodium in einer Konzentration von 5 bis 1000 Gew.-ppm, bezogen auf das homogene Reaktionsgemisch einsetzt.

7. Verfahren gemäß Anspruch 6, **dadurch gekennzeichnet, dass** man Rhodium in einer Konzentration von 10 bis 700 Gew.-ppm, vorzugsweise von 20 bis 500 Gew.-ppm, bezogen auf das homogene Reaktionsgemisch, einsetzt.

8. Verfahren gemäß einem oder mehreren der Ansprüche 1 bis 7, **dadurch gekennzeichnet, dass** das molare Verhältnis von Rhodium zu Phosphor 1 : 5 bis 1 : 200 beträgt.

9. Verfahren gemäß Anspruch 8, **dadurch gekennzeichnet, dass** das molare Verhältnis von Rhodium zu Phosphor 1 : 10 bis 1: 100 beträgt.

10. Verfahren gemäß einem oder mehreren der Ansprüche 1 bis 9, **dadurch gekennzeichnet, dass** man bei der Hydroformylierung bei Temperaturen von 80 bis 150°C, vorzugsweise von 90 bis 140°C, und bei Drücken von 10 bis 30 MPa, vorzugsweise von 20 bis 30 MPa, arbeitet.

11. Verfahren gemäß einem oder mehreren der Ansprüche 1 bis 10, **dadurch gekennzeichnet, dass** man die reduktive Aminierung in Gegenwart von Nickelkatalysatoren bei Temperaturen von 60 bis 150°C und bei einem Wasserstoffdruck von 2 bis 12 MPa durchführt.

12. Verfahren gemäß Anspruch 11, **dadurch gekennzeichnet, dass** man zur reduktiven Aminierung das aus 3(4),7(8)-Bisformyl-bicyclo[4.3.0]nonan und einem primären Amin mit 2 bis 10 Kohlenstoffatomen im Molekül gebildete Diazomethin einsetzt.

13. Die chemische Verbindung 3(4),7(8)-Bis(aminomethyl)-bicyclo[4.3.0]nonan.
